# EUROPEAN PATENT SPECIFICATION

(11) **EP 1 639 136 B1**
(45) Date of publication and mention of the grant of the patent: **13.02.2008**
(21) Application number: 04753239.5
(22) Date of filing: 22.05.2004
(51) Int. Cl.: C12Q 1/68

(54) **FLUORESCENCE-BASED DNA PRIMASE ASSAYS**
FLUORESZENZ-BASIERTE ASSAYS VON DNS-PRIMASEN
DOSAGES D'ADN PRIMASES PAR FLUORESCENCE

(30) Priority: 23.05.2003 US 473054 P; 23.05.2003 US 472967 P
(43) Date of publication of application: 29.03.2006
(73) Proprietor: AstraZeneca AB, 151 85 Södertälje (SE)
(72) Inventor: CHEN, Guo Qiang, AstraZeneca R & D Boston, Waltham, MA 02451 (US); LIU, CE Feng, AstraZeneca R & D Boston, Waltham, MA 02451 (US)
(86) International application number: PCT/US2004/016376
(87) International publication number: WO 2004/106556

(56) References cited:
- WO-A-00/58270
- WO-A-98/59044
- SWART J R ET AL: "PRIMER SYNTHESIS KINETICS BY ESCHERICHIA COLI PRIMASE ON SINGLE- STRANDED DNA TEMPLATES" BIOCHEMISTRY, AMERICAN CHEMICAL SOCIETY. EASTON, PA, US, vol. 34, 1995, pages 16097-16106, XP002911364 ISSN: 0006-2960
- FRICK D N ET AL: "Interaction of bacteriophage T7 gene 4 primase with its template recognition site." THE JOURNAL OF BIOLOGICAL CHEMISTRY. 10 DEC 1999, vol. 274, no. 50, 10 December 1999 (1999-12-10), pages 35889-35898, XP002309417 ISSN: 0021-9258

## Description

### FIELD OF THE INVENTION

The present invention relates to methods for assaying DNA primase activity and methods for identifying compounds that modulate DNA primase activity.

### BACKGROUND

DNA primase (primase) is an enzyme that catalyses the synthesis of short RNA primers on a DNA template during DNA replication (Frick et al., 2001, Annu. Rev. Biochem., 70:39-80). At the DNA replication fork, the parent double-stranded DNA is unwound by helicase (DnaB). DNA primase (DnaG) uses the single-stranded DNA as a template and NTPs as substrates to synthesize RNA primers. These RNA primers are typically 10 - 12 nucleotides long, and are used by DNA polymerase to synthesize the complementary strand of the parent DNA. *In vitro* experiments have demonstrated that the primase activity is significantly stimulated by helicase (Lu et al., 1996, Proc. Natl. Acad. Sci. USA, 93:12902-12907). Helicase not only increases the primase-DNA binding affinity, but also stimulates primase catalytic activity (Johnson et al., 2000, Biochemistry, 39:736-744). The critical functions of primases in DNA replication processes make these enzymes attractive targets for therapeutic intervention in infectious disease and cancer.

In the area of infectious disease, primase presents an excellent antibiotic target for the development of antibiotic compounds and pharmaceuticals for a number of reasons: 1) primase plays key roles in DNA replication and is essential in all bacteria; 2) there are significant structural differences between mammalian and bacterial primases; and 3) three-dimensional (3-D) crystal structure information is available for many bacterial primases.

Assays of DNA primase activity are important for the identification of compounds that modulate primase and that can be used for therapy. Assays of DNA primase activity have been described that are based upon the incorporation of detectably labeled nucleoside triphosphates into a polymerization product and/or immobilization and subsequent detection of a polymerization product (Johnson et al., 2000, Biochem., 39:736-744; Earnshaw & Pope, 2001, J. Biomol. Screen., 6:39-46; Zhang et al., 2002, Anal. Biochem., 304:174-179; US Patent No. 6,043,038; US Patent No. 6,096,499; WO 98/59044).

### SUMMARY

The present invention provides methods for assaying DNA primase activity comprising contacting a nucleic acid template, a DNA primase, and ribonucleoside triphosphates, polymerizing the triphosphates to form RNA, and detecting the RNA with a fluorescent marker that binds the RNA, wherein the detecting step requires no further steps of separating the RNA product from the incubated reaction mix.

The invention also provides methods for identifying compounds that modulate DNA primase activity comprising contacting a nucleic acid template, a DNA primase, and ribonucleoside triphosphates, with a test compound, polymerizing the triphosphates to form RNA, binding a fluorescent marker to the RNA, and detecting a fluorescent signal such that the RNA is directly detected, wherein the detecting step requires no further steps of separating the RNA product from the incubated reaction mix and wherein a change in the fluorescent signal in the presence of the compound as compared with the fluorescent signal in the absence of the compound indicates that the compound modulates DNA primase activity.

### BRIEF DESCRIPTION OF THE DRAWINGS

Figure 1 is a line graph depicting the linear relationship between the fluorescent signal and RNA concentration.
Figure 2 is a line graph depicting linearity of primase activity (fluorescence) with time.
Figure 3 is a line graph depicting linearity between the primase reaction rate and primase concentration.
Figure 4 is a line graph depicting the effect of helicase concentration on primase activity.
Figure 5 is a line graph depicting the effect of DNA template concentration on primase activity.
Figure 6 is a line graph depicting the effect of NTP concentration on primase activity.
Figure 7 is a line graph depicting the effect of magnesium concentration on primase activity.
Figure 8 is a line graph depicting the effect of NaCl concentration on primase activity, in the presence and in the absence of NTPs.
Figure 9 is a bar graph depicting the effect of salt composition on primase activity. "-NTP" represents the control in which no NTP is added. "+NTP" indicates that NTPs were added to the reaction. A, no additional salt was added; B, ammonium chloride; C, ammonium sulfate; D, sodium sulfate; E, potassium chloride; F, sodium acetate; G, calcium chloride.
Figure 10 is a line graph depicting the effect of DMSO concentration on primase activity.
Figure 11 is a plot depicting the effect of pH on primase activity.
Figure 12 is a line graph depicting the effect of temperature on primase activity.
Figure 13 is a bar graph depicting the effect of freeze-thaw cycles on primase (DnaG) and helicase (DnaB) activity.
Figure 14 is a line graph depicting the effect of storage conditions (on ice or at room temperature) on primase activity.
Figure 15 is a line graph depicting inhibition of primase activity by ddATP.
Figure 16 is an image of a SYBR Green II-stained gel of electrophoresed primase reaction products.

### DETAILED DESCRIPTION

The present invention provides methods for assaying the activity of DNA primases, and methods for screening for compounds that modulate the activity of DNA primases. The present invention is based in part upon our discovery that fluorescent markers can be effectively used to monitor DNA primase activity by detection of the RNA product.

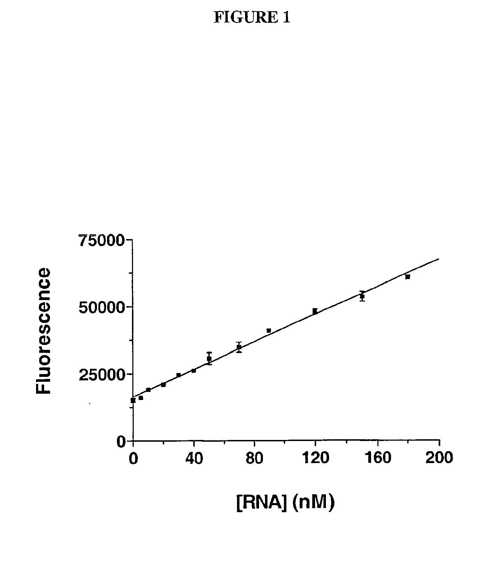

The assays of the present invention are based upon the interaction between RNA and a fluorescent reagent that generates a fluorescent signal. We have found that that there is a linear relationship between the fluorescent signal and the concentration of RNA synthesized by DNA primase even in the presence of the DNA template (Figure 1). Thus, it is not necessary to remove the template or to isolate the RNA product. This finding can be harnessed in assays of DNA primase activity, via the direct measurement of RNA formation, and in assays to identify compounds that modulate these enzymes.

Assays of the present invention exploit the feature that only the polymerized polynucleotide product generates a fluorescent signal while the free (unincorporated) substrate NTPs do not. Therefore, the fluorescence-based assays of the present invention do not require additional procedures to separate the RNA product from the substrate NTPs. Assays of the present invention do not require the use of a solid phase or separation procedure, do not require coupling to enzymatic or immunological reactions, and do not require any immobilization steps. Since the fluorescence-based assays of the present invention do not depend on complicated inter-phase interactions or coupled protein functions, they provide highly specific measurement of DNA primase activity.

Assays of the present invention are amenable to high-throughput screening (HTS) formats.

RNA polymerases constitute a variety of enzymes having similar functions and properties, in particular, the ability to synthesize RNA. DNA primases represent a class of RNA polymerase.

One aspect of the present invention relates to methods for assaying the activity of DNA primase. These assays can be used to track or monitor enzyme activity, to detect enzyme activity in a sample, and for the assessment of nucleic acid template quality or ability to support enzyme activity.

The assays of DNA primase activity comprise combining a nucleic acid template, a DNA primase, and ribonucleoside triphosphates, under conditions in which the RNA polymerase or DNA primase is catalytically active and polymerizes the triphosphates to form RNA. The RNA is contacted with a fluorescent marker that binds the RNA, and a fluorescent signal is detected.

Another aspect of the present invention relates to methods for screening for modulators of DNA primase activity. The modulator screening assays comprise combining, in the presence and in the absence of a test compound, a nucleic acid template, a DNA primase, and ribonucleoside triphosphates under conditions in which the RNA polymerase or DNA primase is catalytically active and polymerizes the triphosphates to form RNA. The RNA is contacted with a fluorescent marker that binds RNA, and a fluorescent signal is detected. The fluorescent signal detected in the presence of the test compound is compared to the fluorescent signal detected in the absence of the test compound. A change or alteration in the fluorescent signal detected in the presence of the compound compared to the fluorescent signal detected in the absence of the compound is indicative of a compound that modulates DNA primase activity.

As used herein, the terms "modulate" or "modulates" in reference to DNA primase activity includes any measurable alteration, either an inhibition or enhancement, of DNA primase activity.

Any DNA primase can be used in the assays of the present invention. Primases have been identified in prokaryotes, including bacteria, bacteriophage, eukaryotes, and eukaryotic viruses.

Many bacterial primase nucleotide and amino acid sequences are known, including *Bacillus subtilis* (X03897), *Clostridium acetobutylicum* (Z23080), *Escherichia coli* (J01687), *Haemophilus influenzae* (L11044), *Helicobacter pylori* (AE000523), *Lactococcus lactis* (D10168), *Legionella pneumophila* (U63641), *Listeria monocytogenes* (U13165), *Myxococcus xanthus (U20669), Mycoplasm genitalium (U39703), Mycoplasma pneumoniae* (1674174), *Mycobacterium tuberculosis* (Z83860), *Pseudomonas putida* (U85774), *Rickettsia prowazekii* (M95860), *Salmonella typhimurium* (M14427), *Staphylococcus aureus* (AB001896), *Synechococcus elongatus* (PCC7942), *Cyanobacteria chroococcales* (X94247), Synechocystis sp. (D90912), *Cyanobacteria chroococcales* (D90912). Additonal bacterial primases for which nucleotide and amino acid sequences are known include *Aquifex aeolicus* (AE000743), *Bacillus stearothermophilus* (AF106033), *Borrelia burgdorferi* (AE001171), *Buchnera aphidicola* (P32000), *Campylobacter jejuni* (CAB73626), *Chlamydia trachomatis* (3329259), *Chlamydophila pneumoniae* (AE001674), *Deinococcus radiodurans* (AAF10180), *Mycobacterium smegmatis* (AF027507), *Neisseria meningitidis* (CABS4964), *Streptomyces coelicolorA3(2)* (CAB51551), *Thermotoga maritima* (AAD36520), *Treponema pallidum* (3322781).

Bacteriophage primases for which nucleotide and amino acid sequences are known include, for example, T7 (gene 4 protein) (P03692), T3 (gene 4 protein) (P20315), P4 (α protein) (PRBPP4), and T4 (gene 41 protein) (ADD42502).

Herpes simplex virus type 1 is the prototype for the herpesvirus family. UL5, UL8, and UL52 proteins compose the heterotrimeric primase/helicase enzyme from this virus. Herpesviral UL52 primases include Human type 1, alphaherpesvirus (Acc# P10236), Equine type 1, alphaherpesvirus (Acc# M86664:7064..10301), Equine type 4, alphaherpes virus (Acc# AF030027:6658..9900), Bovine type 1, alphaherpesvirus (Acc# AJ004801:4013..7237), Human type 3 = varicella-zoster virus, alphaherpesvirus (Acc# P09270), Pseudorabies type 1 = Suid herpesvirus, alphaherpesvirus (Acc# X87246:1087..3963), Human type 7, betaherpesvirus (Acc# AF037218:68725..71310), Human type 6, betaherpes virus (Acc# P52540), Equine type 2, betaherpesvirus (Acc# S55651), Saimiriine type 2, gammaherpes virus (Acc# M86409:6378..8885), Human type 8 = Kaposi's herpesvirus, gammaherpesvirus (Acc# U93872:79735..82266), Wildebeest type 1 = Alcelaphine herpesvirus, gammaherpesvirus (Acc# AF005370:81523..54336), Human type 4 = Epstein-Barr virus, gammaherpes virus (Acc# P03193), Human Cytomegalovirus, betaherpesvirus (Acc# P17149), Mouse Cytomegalovirus, betaherpesvirus (Acc# L07319:3153..6047).

Eukaryotic primase activity is typically associated with two types of multimeric complex. Primases involved in DNA replication are generally found in a complex with DNA polymerase α; these complexes typically include two primase subunits (Pril (49 kDa) and Pri2 (58 kDa)), as well as DNA polymerase α and a p70-90 subunit. Primase activity is also found as a heterodimer which consists of the Pri1 and Pri2 subunits. The active site for RNA polymer elongation is found in the Pri1 subunit, while initiation generally involves the Pri2 subunit. Amino acid and nucleotide sequences for several eukaryotic primase Pri 1 subunits have been characterized, including human (Swissprot Accession No. P49642, Genbank X74330), *Mus musculus* (GenBank J04620), *Rattus norvegicus* PID:g1763025, *Drosophila melanogaster* p50 (PID:g666989), *Caenorhabditis elegans* p48 (SP:P34471), *Saccharomyces cerevisiae* p48 (SP:P10363), *Schizosaccharomyces pombe* p53 (Acc# Z98531:22398 ... 23846), *Plasmodium falciparum* p53 (Acc# X99254:486 ... 3750). Archaeobacteria having primases that are similar to the eukaryotic Pri1 include *Methanococcus jannaschii* (Acc# Q58249), *Archaeoglobus fulgidus* (Acc# AE001054:8352 ... 9434), and *Methanobacterium thermoautotrophicum* (Acc# AE000840:7684 ... 8655). Pri2 primases have been sequenced from human Acc# P49643, *Mus musculus* Acc# S45629, *Caenorhabditis elegans* (Acc# Z81137) *Saccharomyces cerevisiae* (Acc# P20457) and the plant *Arabidopsis thalians* (Acc# AC002130:39565 ... 46348).

DNA primase can be obtained for use in the present invention according to procedures well known to the art. DNA primase can be obtained by isolation or purification from natural sources or can be expressed using recombinant technology. Primase can be expressed as a single target protein or co-expressed with other proteins. Primase can be expressed with or without peptide tags or fusion proteins. Primase can be isolated as a cell extract, prepared in substantially pure form as a single protein, or prepared as a protein complex. Numerous techniques for obtaining DNA primase proteins, including bacterial DNA primase proteins, have been described in the literature. Catalytically active portions or fragments of DNA primase can also be used in the assays of the present invention. For example, the catalytic domain of DNA primase from *E*. *coli* is found within amino acid residues 111H - 433K, the catalytic domain for *H. influenzae* DNA primase is found within amino acid residues 115T - 434 K, and the catalytic domain for *S. pneumoniae* DNA primase is found within amino acid residues 105S - 455T).

Assays of the present invention are conducted under conditions such that the DNA primase is catalytically active. These are conditions under which the DNA primase is capable of polymerizing the substrate ribonucleoside triphosphates to form RNA. Such conditions are known to those of skill in the art. A wide variety of incubation conditions can be used, depending on the enzyme used. Reaction conditions in which DNA primase is active *in vitro* are exemplified below and/or are otherwise known in the art.

Assays of the present invention can be performed in different formats.

In some embodiments, a high-throughput screening (HTS) format is used. HTS can be run at different temperatures, but it is important to keep a constant temperature for the reaction and controls. In some embodiments, room temperature is used.

In some embodiments, a HTS format is used and the primase reaction is carried out on a HTS assay plate in the presence of helicase. In such formats, relatively low DNA template concentrations are used. In assays where primase modulators are screened in a HTS format in the presence of helicase, hits obtained from the assay can include compounds that directly interact with primase and those that modulate primase activity through interactions with helicase.

In some embodiments of the present invention, a premix containing DNA primase, helicase, a DNA template, and the reaction buffer is prepared for use in HTS formats of the assays. It is recommended to prepare the premix freshly for each HTS run. For short time storage, the premix should be placed on ice.

In some embodiments, a HTS format is used and the primase reaction is carried out on a HTS assay plate in the absence of helicase. In such formats, relatively high DNA primer concentrations are used.

In some embodiments of the present invention, non-HTS formats are used.

It is also described that the primase reaction product is isolated or separated from the DNA template before detection. The DNA template can be removed by many procedures known to those of skill in the art, including, but not limited to, electrophoresis, chromatography or using enzymatic reactions. Isolation of the RNA product is useful for characterizing the primase reaction.

In the assays of the present invention, the RNA is detected by using a fluorescent marker that binds RNA. In some embodiments, the detecting is accomplished by measuring the intensity of the fluorescence generated by the interaction of the fluorescent marker and the RNA.

Any fluorescent dye or marker that interacts with RNA can be used in the assays of the present invention. Commercially available fluorescent dyes can be found in a Molecular Probes catalog. For example, RiboGreen, SYBR Green II, and YO-PRO-1 can be used to detect RNA molecules. The fluorescent reagent SYBR Green II is about 4 times more sensitive to RNA than DNA. In addition, the fluorescent background due to DNA can be subtracted from the assay. Fluorescent signals and intensity can be detected with standard fluorescence readers or scanners known to the art. The fluorescent dye or marker can be included during the enzymatic reaction (RNA synthesis) or it can be added after. Where the dye or marker is added after the enzymatic reaction, further incubation is not necessary.

Assays of the present invention utilize nucleic acids as templates for the DNA primase. DNA primase utilizes a nucleic acid template and synthesizes a complementary RNA primer. Templates of any sequence that provides a substrate to which the primase can bind, can be used in the assays of the present invention.

The nucleic acid template used in the primase assays of the invention can be linear or circular, partially or fully double-stranded DNA, depending on source, convenience, and the specificity of the targeted DNA primase. While in particular embodiments, the template material is DNA, other nucleic acids or structural analogs may be substituted so long as they provide an active substrate for the targeted DNA primase activity. The nucleic acids templates can be of any length sufficient to provide a substrate for the primase, a template for assay-detectable nascent primer(s), and that is otherwise amenable to the assay conditions and requirements. The nucleic acid templates can be labeled or non-labeled, modified or non-modified.

In some embodiments, the following nucleic acid templates are used:
5'-(CT)₁₇GCAAAGC-3' (SEQ ID NO:1)
5'-(CT)₁₆GCAAAGC-3' (SEQ ID NO:2)
5'-(CT)₁₆ACC(CT)₃-3' (SEQ ID NO:3)
5'-(CT)₁₆GAAAATC-3' (SEQ ID NO:4)
5'-(CT)₁₆GTAAAAC-3' (SEQ ID NO:5)
5'-(CT)₁₆GGAAACC-3' (SEQ ID NO:6)
5'-(CT)₁₆AGT(CT)₃-3' (SEQ ID NO:7)
5'-(CT)₁₆CCAAAGG-3' (SEQ ID NO:8)

In some embodiments, the nucleic acid template of SEQ ID NO:1, SEQ ID NO:2, or SEQ ID NO:8 is used in assays of *E. coli* DNA primase.

In some embodiments, the nucleic acid template of SEQ ID NO:3 or SEQ ID NO:7 is used in assays of *S. aureus* DNA primase.

In some embodiments, the nucleic acid template of SEQ ID NO:3 is used in assays of *S. pneumoniae* DNA primase.

In some embodiments, the nucleic acid template of SEQ ID NO:2, 4, 5, or 6 is used in assays of *H. influenzae* DNA primase.

DNA templates can be provided for use in the assays of the present invention according to many methods known to the art, including chemical or enzymatic synthesis, and isolation from cells.

Assays of the present invention can optionally be carried out in the presence of replisome proteins such as helicase, single strand DNA binding protein, or DNA polymerase. In some embodiments, helicase is included in the reaction mixture. Although DnaB stimulates primase activity, DnaB is not an essential component in the primase reaction. The fluorescence-based assay does not rely on the presence of DnaB. In some embodiments, DnaB (*E. coli* helicase) is included in the reaction mixture to stimulate primase activity.

Nucleoside triphosphates used in the assays of the present invention can be in modified or unmodified forms, including, but not limited to, radioactively labeled or fluorescence-labeled, or modified for activity or detection purposes.

The invention is further illustrated by way of the following examples, which are intended to elaborate several embodiments of the invention. These examples are not intended to, nor are they to be construed to, limit the scope of the invention. It will be clear that the invention may be practiced otherwise than as particularly described herein.

### EXAMPLES

### Example 1. Fluorescence-based E. coli DNA primase HTS assay in the presence of helicase.

The reaction was carried out on a Costar 384-well black plate. The reaction volume was 30 µl. The final concentrations in the reaction were 50 mM Tris-HCl, pH 7.5, 10 mM Mg(OAc)₂, 10 mM DTT, 25 mM potassium glutamate, 0.003 % Brij-35, 200 nM DNA, 25 nM helicase, 75 nM *E*. *coli* DNA primase, 200 µM ATP and 200 µM GTP. To perform the HTS assay, 2 µl of compound / DMSO solution or aqueous DMSO solution was incubated with 23 µl of a reaction premix at room temperature for 15 min. The reaction premix was a 1.3 x concentrated reaction solution without NTPs. After the incubation, 5 µl of a substrate solution with 1.2 mM ATP and 1.2 mM GTP were added to initiate the reaction. The reaction was incubated at room temperature for 40 min. Then 25 µl of a 30 X SYBR Green II solution with 1.2 M NaCl was added and the fluorescence was measured (excitation at 485 nm, emission at 535 nm).

### Example 2. Linearity with time and E. coli DNA primase concentration.

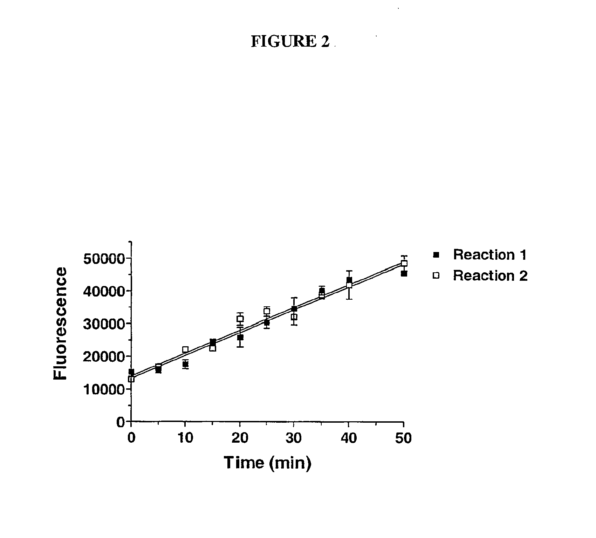

The reaction was linear with time until the signal-to-background ratio reached 3.5 - 4.0. Beyond that point, the free DNA concentration was too low and the reaction linearity was lost. Shortage of NTPs also had an effect on the reaction linearity. Under the HTS reaction conditions, the linear range of time was about 40 min and the signal-to-background ratio was about 3 (Figure 2).

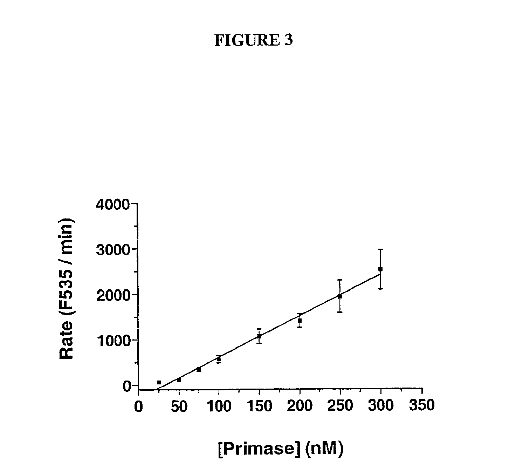

The linearity between fluorescence intensity and primase concentration is shown in Figure 3. A higher primase concentration limited the reaction linearity to a shorter period of time. When the primase concentration was about 25 nM or lower, the reaction signal was negligible within 40 min.

### Example 3. Helicase concentration dependence of E. coli DNA primase activity.

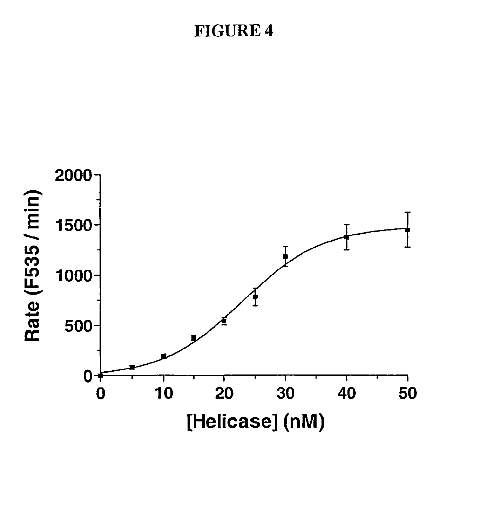

In the absence of helicase, primase activity was negligible at DNA concentrations of 200 nM or lower. Primase activity reached a maximum when the hexameric helicase concentration was about 35 to about 40 nM (Figure 4). Helicase concentrations higher than 25 - 30 nM resulted in a non-linear reaction within 40 min. In addition, high helicase concentrations consumed NTPs quickly.

### Example 4. DNA and NTP concentration dependence E. coli DNA primase activity.

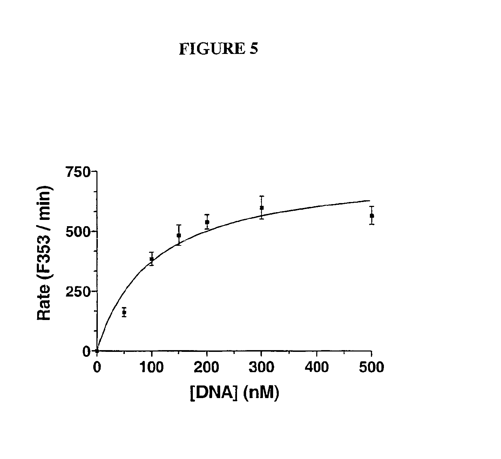

DNA7 (5'-CTCTCTCTCTCTCTCTCTCTCTCTCTCTCTCTCTGCAAAGC-3' (SEQ ID NO:1)) was used as the DNA template in the *E*. *coli* DNA primase assay. The primase reaction rate reached a maximum when the DNA concentration was about 300 nM. The apparent Kₘ for DNA was 105 nM (Figure 5). The DNA concentration in the HTS assay was 200 nM.

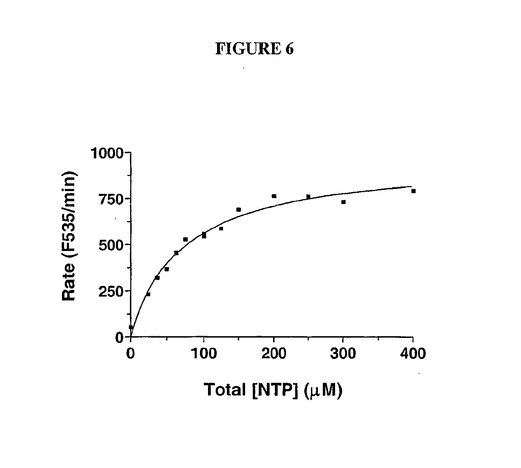

NTPs are not only used by primase to synthesize RNA but are also hydrolyzed by helicase. Under the HTS reaction condition, 25 nM helicase hydrolyzed NTPs at a rate of 4.8 µM / min. Figure 6 shows the dependence of the initial rate of the primase reaction on the total NTP concentration. The apparent Kₘ for NTP was 71 µM. The NTP concentration in the HTS assay was 200 µM.

### Example 5. Magnesium concentration effect on E. coli DNA primase activity.

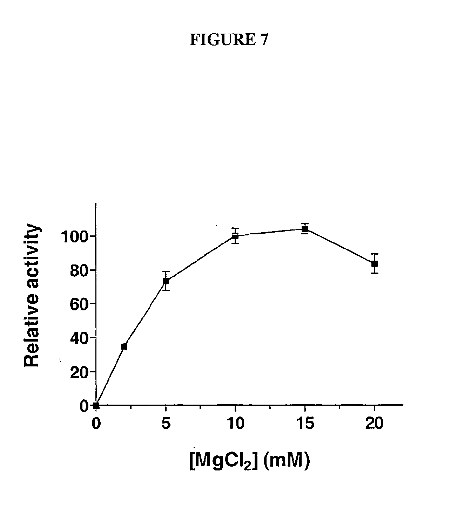

As shown in Figure 7, the optimal magnesium chloride concentration under the HTS assay condition was about 10 to about 15 mM. A higher magnesium concentration inhibited primase activity.

### Example 6. Salt / ionic strength effect on E. coli DNA primase activity.

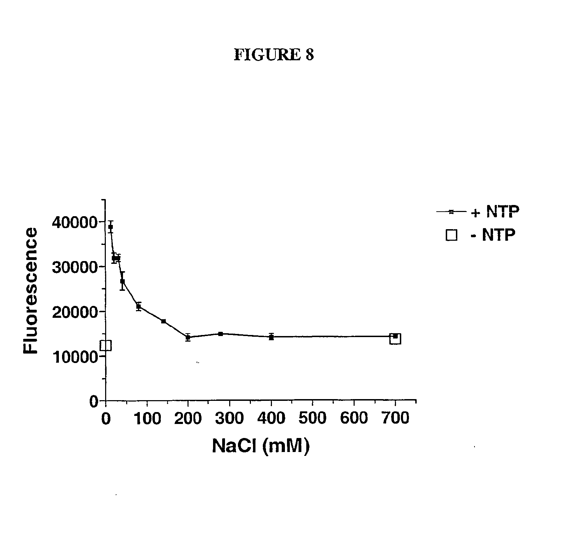

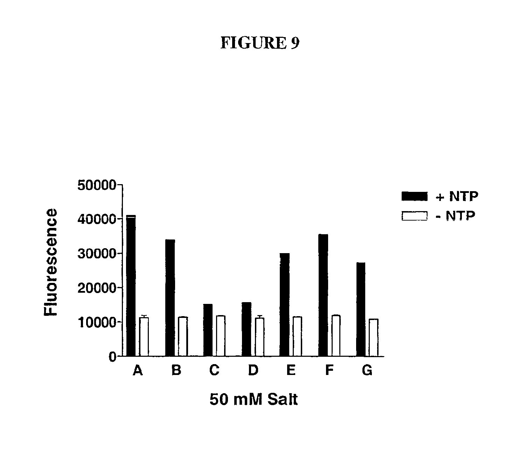

Primase activity was significantly inhibited by high salt concentrations (Figure 8). For example, 50 mM NaCl or KCl inhibited approximately 50 % of primase activity. When the NaCl concentration was 200 mM or higher, primase activity was negligible. In the low salt concentration range, 0 - 5 mM NaCl, primase reactivity was stable. Salt composition also affected enzyme activity, as shown in Figure 9.

### Example 7. DMSO tolerance of E. coli DNA primase activity.

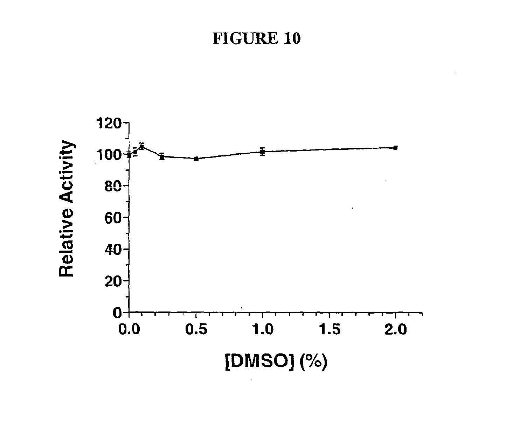

Primase activity was not significantly affected by DMSO at concentrations of about 0 to about 2 %, as shown in Figure 10. DMSO at concentrations of about 0 to about 2 % also did not interfere with the interaction between RNA and SYBR Green II.

### Example 8. pH dependence of on E. coli DNA primase activity.

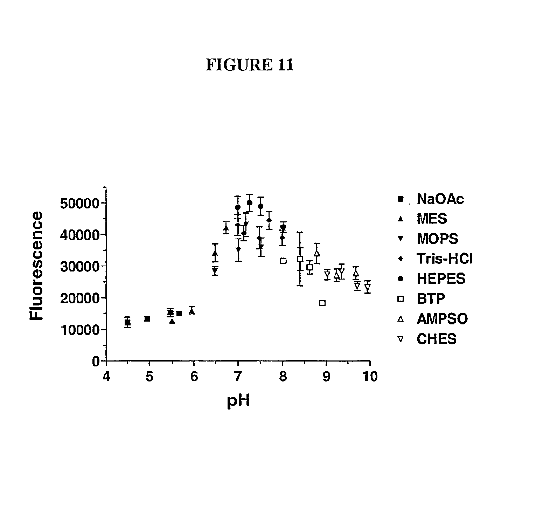

The optimal pH for primase activity was 7.5 - 8. Although the choice of buffers affected enzyme activity to some degree, it was observed that in general a pH higher than 8.5 reduced enzyme activity and a pH lower than 6 virtually abolished it (Figure 11).

### Example 9. Temperature dependence of E. coli DNA primase activity.

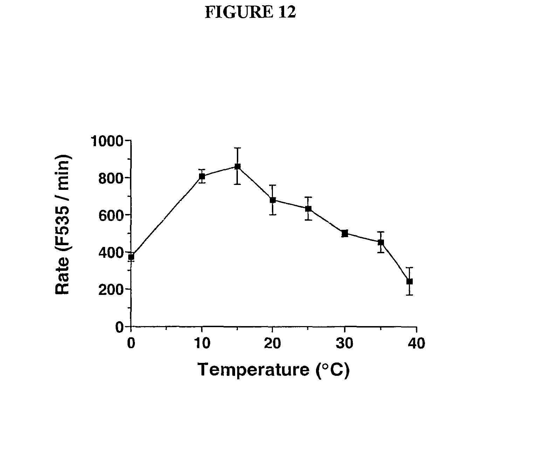

The primase reaction reached a maximum rate at temperatures around 15°C (Figure 12).

### Example 10. Enzyme stability of E. coli DNA primase.

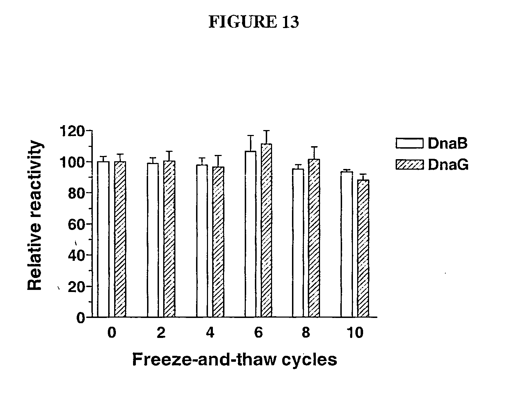

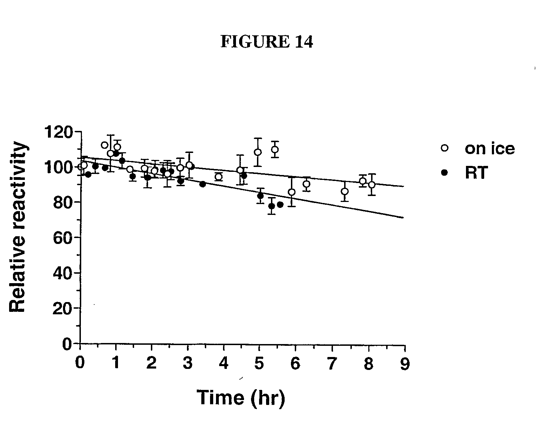

Purified primase and helicase were stable after repeated freeze-and-thaw cycles, as shown in Figure 13. To check the stability of the premix that contained primase, helicase, DNA and the reaction buffer, the solution was incubated on ice or at room temperature (22.5°C) for different periods of time, and then the NTPs were added to start the reactions. The results (Figure 14) show that primase activity decreased at a rate of about 1.7 % per hour on ice and at a rate of about 3.5% per hour at room temperature.

### Example 11. HTS assay validation.

Using a MultiMek 96 robot for addition of reagents into the Costar 384-well plates, window sizes ranging from 10 to 15 were obtained. The inter-plate deviation was about 3 %.

### Example 12. IC₅₀ of ddATP on E. coli DNA primase activity.

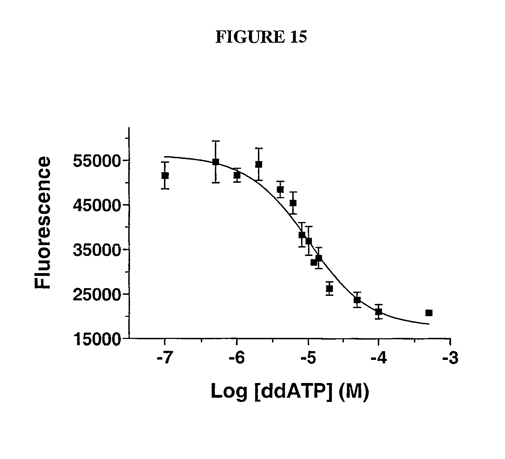

A dideoxy NTP was used as a positive inhibitory control for the primase reaction. Figure 15 shows an IC₅₀ measurement of inhibition by ddATP. Since ddATP competes with NTP (ATP in this case) in the primase reaction, the IC₅₀ value was dependent on the substrate (ATP) concentration. Under the HTS conditions used, the IC₅₀ of ddATP was 10 µM.

### Example 13. Non-HTS format assay with E. coli DNA primase.

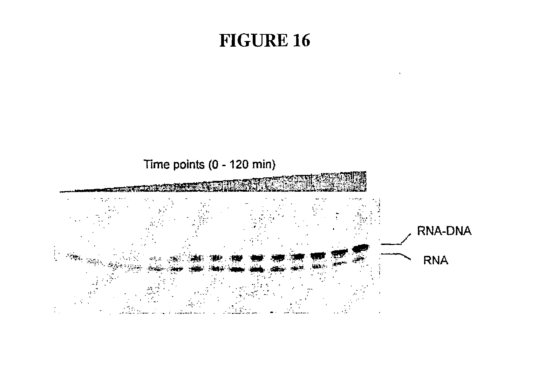

In this format, the reaction product was isolated from the DNA template before detection. The primase reaction solution, with 2 µM DNA template, was loaded onto a polyacrylamide gel. After electrophoresis, the gel was stained with a SYBR Green II solution (Figure 16). The primase product formed RNA-DNA duplex under non-denaturing conditions and was separated from the free DNA template. The primase reaction yield was calculated from the band intensity.

### Example 14. Measurement of DNA primase catalytic domain activity. Cloning of DNA primase catalytic domains

Catalytic domains of DNA primase from *E. coli* (encoding amino acid residues 111H - 433K), *H. influenzae* (encoding amino acid residues 115T - 434 K) and *S*. *pneumoniae* (encoding amino acid residues 105S - 455T) were cloned, expressed in *E. coli* and purified by ion exchange and affinity chromatographic columns.

### E. coli DNA primase catalytic domain

The primase reaction mixture was composed of 10 mM Tris-HCl, pH 8.0, 100 mM KGlu, 10 mM Mg(OAc)₂, 10 mM DTT, 0.005% Brij-35, 10 % glycerol, 2 µM DNA template, 100 µM ATP, 100 µM GTP, 1 µM *E. coli* primase domain. The reaction was carried out at room temperature for 30 min. or a time-course experiment was performed to observe time dependence. To determine the RNA level, 30 µl of the reaction mixture was mixed with 30 µl of 30 x SYBR II and the fluorescence was measured, or the reaction mixture (15 µl) was loaded onto a TBE gel and the gel was stained with 1 x SYBR II after electrophoresis.

### S. pneumoniae primase domain

The primase reaction mixture was composed of 10 mM MES, pH 6.5, 125 mM NH₄Cl, 10 mM Mg(OAc)₂, 10 mM CaCl₂, 1 mM DTT, 0.003% Brij-35, 1 µM DNA template, 200 µM ATP, 200 µM GTP, 0.5 µM *S*. *pneumoniae* primase domain. The reaction was carried out at room temperature for 30 min., or a time-course experiment was performed to observe time dependence. To determine the RNA level, 30 µl of the reaction mixture was mixed with 30 µl of 30 x SYBR II and the fluorescence was measured, or the reaction mixture (15 µl) was loaded onto a TBE gel and the gel was stained with 1 x SYBR II after electrophoresis.

### H. influenzae primase domain

The primase reaction mixture was composed of 10 mM Tris-HCl, pH 7.5, 125 mM NH₄Cl, 10 mM Mg(OAc)₂, 5 mM CaCl₂, 1 mM DTT, 0.003% Brij-35, 2 µM DNA template, 200 µM ATP, 200 µM GTP, 0.2 µM *H. influenzae* primase domain. The reaction was carried out at room temperature for 30 min., or a time-course experiment was performed to observe time dependence. To determine the RNA level, 30 µl of the reaction mixture was mixed with 30 µl of 30 x SYBR II and the fluorescence was measured, or the reaction mixture (15 µl) was loaded onto a TBE gel and the gel was stained with 1 x SYBR II after electrophoresis.

The foregoing examples are meant to illustrate the invention and are not to be construed to limit the invention in any way.

## Claims

1. A method for assaying DNA primase activity comprising
providing a reaction mix comprising a nucleic acid template, a DNA primase, and ribonucleoside triphosphates;
incubating the reaction mix such that the triphosphates polymerize to form RNA ; and
detecting directly the RNA with a fluorescent marker that binds the RNA, wherein the detecting step requires no further steps of separating the RNA product from the incubated reaction mix.

2. A method for identifying compounds that modulate DNA primase activity comprising
contacting a nucleic acid template, a DNA primase, and ribonucleoside triphosphates, with a test compound;
polymerizing the triphosphates to form RNA;
binding a fluorescent marker to the RNA; and
detecting a fluorescent signal such that the RNA is directly detected, wherein the detecting step requires no further steps of separating the RNA product from the incubated reaction mix and wherein a change in the fluorescent signal in the presence of said compound as compared with the fluorescent signal in the absence of said compound indicates that said compound modulates DNA primase activity.

3. The method according to claim 1 or 2, wherein the fluorescent marker is added before polymerization.

4. The method according to claim 1 or 2, wherein the fluorescent marker is added after polymerization.

5. The method according to claim 1 or 2, wherein the DNA primase is bacterial.

6. The method according to claim 1 or 2, wherein the DNA primase is selected from *E. coli* DNA primase, *S. aureus* DNA primase, *S. pneumoniae* DNA primase, and *H. influenzae* DNA primase.

7. The method according to claim 1 or 2, wherein the fluorescent marker is selected from SYBR Green II, RiboGreen, and YO-PRO-1.

8. The method according to claim 1 or 2, wherein the nucleic acid template is selected from SEQ ID NO:1, SEQ ID NO:2, SEQ ID NO:3, SEQ ID NO:4, SEQ ID NO:5, SEQ ID NO:6, SEQ ID NO:7, and SEQ ID NO:8.

9. The method according to claim 1 or 2, wherein the assay is carried out in the presence of helicase.

10. The method according to claim 1 or 2, wherein the detecting is accomplished by measuring fluorescence intensity.

## Patentansprüche

1. Verfahren für einen Assay der DNA-Primase-Aktivität, umfassend
Bereitstellen eines Reaktionsgemischs, das eine Nukleinsäurematrize, eine DNA-Primase und Ribonukleosidtriphosphate umfasst;
Inkubieren des Reaktionsgemischs derart, dass die Triphosphate unter Bildung von RNA polymerisieren, und
direktes Nachweisen der RNA mit einem fluoreszierenden Marker, der die RNA bindet, wobei der Nachweisschritt keine weiteren Schritte der Abtrennung des RNA-Produkts aus dem inkubierten Reaktionsgemisch erfordert.

2. Verfahren zur Identifikation von Verbindungen, welche die DNA-Primase-Aktivität modulieren, umfassend
Zusammenbringen einer Nukleinsäurematrize, einer DNA-Primase und von Ribonukleosidtriphosphaten mit einer Testverbindung;
Polymerisieren der Triphosphate unter Bildung von RNA;
Binden eines fluoreszierenden Markers an die RNA und
Nachweisen des Fluoreszenzsignals derart, dass die RNA direkt nachgewiesen wird, wobei der Nachweisschritt keine weiteren Schritte der Abtrennung des RNA-Produkts aus dem inkubierten Reaktionsgemisch erfordert und wobei eine Veränderung im Fluoreszenzsignal in Anwesenheit dieser Verbindung verglichen mit dem Fluoreszenzsignal in Abwesenheit dieser Verbindung anzeigt, dass die Verbindung die DNA-Primase-Aktivität moduliert.

3. Verfahren nach Anspruch 1 oder 2, wobei der fluoreszierende Marker vor der Polymerisation zugegeben wird.

4. Verfahren nach Anspruch 1 oder 2, wobei der fluoreszierende Marker nach der Polymerisation zugegeben wird.

5. Verfahren nach Anspruch 1 oder 2, wobei die DNA-Primase bakteriell ist.

6. Verfahren nach Anspruch 1 oder 2, wobei die DNA-Primase aus *E.-coli*-DNA-Primase, *S.*-*aureus*-DNA-Primase, S.-pneumoniae-DNA-Primase und *H.-influenzae*-DNA-Primase ausgewählt ist.

7. Verfahren nach Anspruch 1 oder 2, wobei der fluoreszierende Marker aus SYBR Green II, RiboGreen und YO-PRO-1 ausgewählt ist.

8. Verfahren nach Anspruch 1 oder 2, wobei die Nukleinsäurematrize aus SEQ ID NR: 1, SEQ ID NR: 2, SEQ ID NR: 3, SEQ ID NR: 4, SEQ ID NR: 5, SEQ ID NR: 6, SEQ ID NR: 7 und SEQ ID NR: 8 ausgewählt ist.

9. Verfahren nach Anspruch 1 oder 2, wobei der Assay in Anwesenheit von Helicase durchgeführt wird.

10. Verfahren nach Anspruch 1 oder 2, wobei der Nachweis durch Messen der Fluoreszenzintensität erfolgt.

## Revendications

1. Procédé pour doser l'activité d'une ADN primase comprenant les étapes consistant à :
fournir un mélange de réaction comprenant une matrice d'acide nucléique, une ADN primase et des ribonucléosides triphosphates ;
incuber le mélange de réaction de sorte que les triphosphates se polymérisent pour former un ARN ; et
détecter directement l'ARN avec un marqueur fluorescent qui se lie à l'ARN, dans lequel l'étape de détection ne requiert aucune étape supplémentaire de séparation du produit d'ARN à partir du mélange de réaction incubé.

2. Procédé pour identifier des composés qui modulent une activité d'ADN primase comprenant les étapes consistant à :
mettre en contact une matrice d'acide nucléique, une ADN primase et des ribonucléosides triphosphates avec un composé à tester ;
faire que les triphosphates se polymérisent pour former un ARN ;
lier un marqueur fluorescent à l'ARN ; et
détecter un signal fluorescent de sorte que l'ARN soit détecté directement, dans lequel l'étape de détection ne requiert aucune étape supplémentaire de séparation du produit d'ARN à partir du mélange de réaction incubé et dans lequel un changement de signal fluorescent en présence dudit composé, par comparaison avec le signal fluorescent en l'absence dudit composé, indique que ledit composé module l'activité de l'ADN primase.

3. Procédé selon la revendication 1 ou la 2, dans lequel le marqueur fluorescent est ajouté avant la polymérisation.

4. Procédé selon la revendication 1 ou la 2, dans lequel le marqueur fluorescent est ajouté après la polymérisation.

5. Procédé selon la revendication 1 ou la 2, dans lequel l'ADN primase est bactérienne.

6. Procédé selon la revendication 1 ou la 2, dans lequel l'ADN primase est choisie parmi une ADN primase d'E. coli, une ADN primase de S. aureus, une ADN primase de S. pneumoniae et une ADN primase de H. influenzae.

7. Procédé selon la revendication 1 ou la 2, dans lequel le marqueur fluorescent est choisi parmi les SYBR Green II, RiboGreen et YO-PRO-1.

8. Procédé selon la revendication 1 ou la 2, dans lequel la matrice d'acide nucléique est choisie parmi les SEQ ID n° : 1, SEQ ID n° : 2, SEQ ID n° : 3, SEQ ID n° : 4, SEQ ID n° : 5, SEQ ID n° : 6, SEQ ID n° : 7 et SEQ ID n° : 8.

9. Procédé selon la revendication 1 ou la 2, dans lequel le dosage est exécuté en présence d'une hélicase.

10. Procédé selon la revendication 1 ou la 2, dans lequel la détection est accomplie en mesurant l'intensité de la fluorescence.


### SEQUENCE LISTING

<110> Astrazeneca AB
<120> Flouresence-based DNA Primase Assays
<130> 100998-1 wo
<140> PCT/US 04/016376
   <141> 2004-05-22
<150> US 60/473,054
   <151> 2003-05-23
<150> US 60/472,967
   <151> 2003-05-23
<160> 8
<170> PatentIn version 3.3
<210> 1
   <211> 41
   <212> DNA
   <213> Artificial Sequence
<220>
   <223> DNA primase template
<400> 1
   ctctctctct ctctctctct ctctctctct ctctgcaaag c 41
<210> 2
   <211> 39
   <212> DNA
   <213> Artificial Sequence
<220>
   <223> DNA primase template
<400> 2
   ctctctctct ctctctctct ctctctctct ctgcaaagc 39
<210> 3
   <211> 41
   <212> DNA
   <213> Artificial Sequence
<220>
   <223> DNA primase template
<400> 3
   ctctctctct ctctctctct ctctctctct ctaccctctc t 41
<210> 4
   <211> 39
   <212> DNA
   <213> Artificial Sequence
<220>
   <223> DNA primase template
<400> 4
   ctctctctct ctctctctct ctctctctct ctgaaaatc 39
<210> 5
   <211> 39
   <212> DNA
   <213> Artificial Sequence
<220>
   <223> DNA primase template
<400> 5
   ctctctctct ctctctctct ctctctctct ctgtaaaac 39
<210> 6
   <211> 39
   <212> DNA
   <213> Artificial Sequence
<220>
   <223> DNA primase template
<400> 6
   ctctctctct ctctctctct ctctctctct ctggaaacc 39
<210> 7
   <211> 41
   <212> DNA
   <213> Artificial Sequence
<220>
   <223> DNA primase template
<400> 7
   ctctctctct ctctctctct ctctctctct ctagtctctc t 41
<210> 8
   <211> 39
   <212> DNA
   <213> Artificial Sequence
<220>
   <223> DNA primase template
<400> 8
   ctctctctct ctctctctct ctctctctct ctccaaagg 39